**Europäisches Patentamt**

**European Patent Office**

**Office européen des brevets**

(19)

(11) Veröffentlichungsnummer: **0 264 636 B1**

(12) **EUROPÄISCHE PATENTSCHRIFT**

(45) Veröffentlichungstag der Patentschrift:
27.12.90

(21) Anmeldenummer: 87113728.7

(22) Anmeldetag: 05.10.85

(60) Veröffentlichungsnummer der früheren Anmeldung
nach Art. 76 EPÜ: 0177912

(51) Int. Cl.⁵: **C07C 29/17, C07C 31/10**

(54) Verfahren zur Herstellung von Propanol.

(30) Priorität: 12.10.84 DE 3437429
01.12.84 DE 3443966

(43) Veröffentlichungstag der Anmeldung:
27.04.88 Patentblatt 88/17

(45) Bekanntmachung des Hinweises auf die Patenterteilung:
27.12.90 Patentblatt 90/52

(84) Benannte Vertragsstaaten:
BE CH DE FR GB IT LI NL

(56) Entgegenhaltungen:
DE-A- 2 145 297
DE-A- 2 536 273
DE-B- 1 285 992
DE-B- 1 668 187
US-A- 4 384 147

**Die Akte enthält technische Angaben, die nach dem Eingang der Anmeldung eingereicht wurden und die nicht in dieser Patentschrift enthalten sind.**

(73) Patentinhaber: BASF Aktiengesellschaft,
Carl-Bosch-Strasse 38, D-6700 Ludwigshafen(DE)

(72) Erfinder: Mueller, Herbert, Dr., Carostrasse 53,
D-6710 Frankenthal(DE)
Erfinder: Reiss, Wolfgang, Dr., Bannwasserstrasse 70,
D-6700 Ludwigshafen(DE)

## Beschreibung

Diese Erfindung betrifft ein Verfahren zur Herstellung von Propanol durch Hydrierung von Propinol.

Alkanole, wie Butandiol-1,4, lassen sich durch katalytische Hydrierung von Alkinolen, wie Butindiol-1,4, herstellen. Bei diesem z.B. in der DE-PS 890 944 oder der US-PS 4 153 578 beschriebenen Verfahren verwendet man z.B. Nickel- oder Kobaltkatalysatoren, die entweder als Raney-Metalle oder als Trägerkatalysatoren eingesetzt werden. Auch Trägerkatalysatoren, die neben Nickel noch Kupfer, Aluminium und Mangan enthalten, sind schon für die Hydrierung von Acetylenalkoholen vorgeschlagen worden (s. DE-OS 2 145 297, US-PS 3 449 445, DE-OS 2 536 273 und DE-OS 2 004 611).

Für die Hydrierung der Alkinole werden die Katalysatoren in suspendierter oder fest angeordneter Form eingesetzt. Man unterscheidet die einstufige Hydrierung, bei der die Hydrierung in einem einmaligen Durchgang durch einen Reaktor erfolgt, und die zweistufige Arbeitsweise. Beim Zweistufen-Prozeß (US-PS 4 153 578) wird in der ersten Stufe bei niederen Drücken und tiefer Temperatur unvollständig hydriert. Ein Teil des Alkinols oder der im Zulauf enthaltenen Carbonylverbindungen bleiben unumgesetzt. Erst in der Nachreaktionszone, in der bei Wasserstoffdrücken über 200 bar und Temperaturen über 100°C gearbeitet wird, findet die vollständige Hydrierung statt.

Es bestand die Aufgabe, für die Hydrierung von Propinol ein Verfahren zu finden, das nicht nur einen hohen Durchsatz ermöglicht, sondern auch den Nachteil hoher Katalysatorkosten, die durch die begrenzte Standzeit von Trägerkatalysatoren oder die hohen Herstellkosten von Vollkatalysatoren verursacht werden, vermeidet. Gleichzeitig sollte es sehr reines Propanol liefern und die Hydrierung in guten Ausbeuten ermöglichen.

Nach dem neuen Verfahren, das diese Aufgabe erfüllt, stellt man Propanol durch katalytische Hydrierung von Propinol in wäßrigem Medium und in Gegenwart eines Hydrierkatalysators bei Drücken über 20 bar Wasserstoffpartialdruck dadurch her, daß man die Hydrierung bei Temperaturen von über 200°C vornimmt.

Das erfindungsgemäße Verfahren zeichnet sich insbesondere dadurch aus, daß es die Hydrierung von Propinol zu Propanol in praktisch quantitativer Ausbeute ermöglicht und dabei ein Endprodukt von hoher Reinheit liefert.

Das Propanol ist frei von Carbonylfunktionen oder Spuren von olefinisch ungesättigten Verbindungen. Das neue Verfahren zeichnet sich durch eine erhöhte Wirtschaftlichkeit aus, weil es die Gewinnung der bei der Reaktion entstehenden Hydrierwärme bei einem hohen Temperturniveau ermöglicht.

Nach dem erfindungsgemäßen Verfahren hydriert man das Propinol (= Propin-2-ol-1) in wäßrigem Medium. Für das erfindungsgemäße Verfahren sind alle gebräuchlichen Hydrierkatalysatoren geeignet, z.B. die für die katalytische Hydrierung geeigneten Metalle, wie Platin, Palladium, Ruthenium, Nickel, Cobalt oder Kupfer. Bevorzugt werden Katalysatoren, die nur Nickel als katalytisch wirkende Komponente enthalten. Besonders geeignet ist z.B. Raney-Nickel oder Nickel, das durch den Zerfall von Nickel-O-Komplexen oder Nickelformiat gebildet wird. Das aktive Metall kann auch auf einem Träger niedergeschlagen sein. Es sind auch Katalysatoren für das Verfahren einsetzbar, die mehrere der bekannten Hydriermetalle als wirksame Komponenten enthalten. Auch der für die Butindiol-Hydrierung üblicherweise eingesetzte Katalysator aus Nickel, Kupfer und Mangan auf Kiselsäure oder aluminiumoxidhaltigen Trägern ist geeignet. Die Katalysatoren werden vorzugsweise in suspendierter Form eingesetzt. Sie werden z.B. dem wäßrigem Medium vor der Hydrierung in feinverteilter Form zugegeben. Man kann anstelle des metallischen Nickels auch wasserlösliche Nickelsalze einer organischen Säure mit mindestens 2 C-Atomen verwenden. Wasserlösliche Nickelsalze einer organischen Säure mit mindestens 2 C-Atomen, sind z.B. Nickelacetat, Nickelpropionat und Nickelbutyrat. Mit Nickelacetat und Nickelpropionat erhält man besonders vorteilhafte Ergebnisse. Bei dieser Arbeitsweise gibt man zu dem zu hydrierenden wäßrigen Gemisch so viel Nickelsalz, daß es Nickel in einer Konzentration von 0,01 bis 0,2 Gew.% enthält. Die bevorzugte Konzentration an Nickel beträgt 0,05 bis 0,15 Gew.%, bezogen auf die wäßrige Propinol-Lösung. Höhere Konzentrationen sind für die Umsetzung unschädlich, verringern aber die Wirtschaftlichkeit des Verfahrens.

Man hydriert bei Temperaturen von über 200°C, wie bei 210 bis 280°C, vorzugsweise bei 230 bis 260°C und z.B. Drücken zwischen 20 und 300 bar, vorzugsweise zwischen 50 und 250 bar. Drücke, die über den bevorzugten Bereich hinausgehen, bringen gemessen am Aufwand keine merklichen Vorteile. Die Konzentration des Hydrierkatalysators richtet sich nach der gewünschten Reaktionsgeschwindigkeit bei der gewählten Reaktionstemperatur. Sie liegt im allgemeinen mit z.B. 0,05 bis 5 Gew.% wesentlicher niedriger als bei der sonst geübten Hydrierarbeitweise. Trägerfreie Katalysatoren werden bevorzugt, da sie sich durch Dekantieren oder Magnet- und Schichtenfiltration besonders leicht und vollständig abtrennen und auch rückführen lassen.

Die Hydrierung kann in einfachen Reaktionsapparaten, wie Blasensäulen, gerührten Reaktionsbehältern oder Umlaufreaktoren, die z.B. durch Düsen für einen ausreichenden Stoffausgleich und zur Durchmischung des Reaktionsgemisches sorgen, durchgeführt werden.

Bei kontinuierlicher Verfahrensführung, die man z.B in einem gerührten Reaktor oder einem Umlaufreaktor durchführt, wird die Hydrierwärme zweckmäßigerweise dazu verwendet, den Frischzulauf z.B. auf eine Eingangstemperatur von 160 bis 180°C aufzuheizen, ehe er in den Reaktor eintritt. Das Aufheizen erfolgt z.B. über einen außenliegenden Wärmetauscher, der von rückgeführten Reaktionsgemisch durchströmt wird. In dem Maße, in dem die Hydrierung längs der Reaktionszone fortschreitet, erhitzt sich das Reaktionsgemisch auf über 200°C. Die Endtemperatur wird in diesem Falle überwiegend von der Menge des Frischzulaufs und der z.B. für die Dampfgewinnung verwendeten Menge der Hydrierwärme bestimmt.

Wird nicht kontinuierlich hydriert, so empfiehlt sich die sogenannte "halbkontinuierlich" durchgeführte Hydrierung. Dabei wird der Katalysator in einem kleinen Teil des Fertigproduktes suspendiert und vorgelegt. Dann wird in das Reaktionsgefäß bei Reaktionstemperatur und Reaktionsdruck Propinol-Lösung kontinuierlich in dem Maße, wie die Hydrierung stattfindet, eingeleitet. Auf diese Weise wird vermieden, daß Propinol im zeitlichen Verlauf der Hydrierung in überhöhter Konzentration im Reaktionsvolumen auftritt.

Das im Reaktionsgemisch suspendierte Hydriermetall trennt man nach der Hydrierung, z.B. nach an sich üblichen phsikalischen Trennmethoden, wie Zentrifugieren, Sedimentieren oder Filtrieren ab. Die Katalysatoren können erneut in den Reaktionsraum zurückgeführt werden. Sie erhalten ihre ursprüngliche Aktivität über sehr lange Reaktionszeiten bei.

Werden Nickelsalze verwendet, so bildet sich unter den Bedingungen des erfindungsgemäßen Verfahrens metallisches Nickel. Man trennt das im Reaktionsgemisch suspendierte Nickel nach der Hydrierung z.B. nach an sich üblichen physikalischen Trennmethoden, wie Zentrifugieren, Sedimentieren oder Filtrieren ab. Das so zurückgewonnene Nickel kann z.B. durch Behandlung mit der entsprechenden organischen Säure, wie Essigsäure in eine Nickelsalz-Lösung überführt werden, die erneut für das erfindungsgemäße Verfahren herangezogen werden kann. Die dabei auftretenden Katalysatorverluste sind vernachlässigbar.

Nach dem neuen Verfahren, das im Gegensatz zu allen Erfahrungen bei Temperaturen arbeitet, die als ungeeignet für die Reaktion galten, erhält man das Propanol in außerordentlich hoher Reinheit. Die Selektivität beträgt praktisch 100 %. Dieser Befund ist überraschend, da man ein so vorteilhaftes Ergebnis z.B. nicht erhält, wenn man die Hydrierung von Propinol auf herkömmliche Art bei Temperaturen unter 200°C vornimmt. Das günstige Verfahrensergebnis konnte auch deshalb nicht erwartet werden, weil Alkine bekanntlich gegenüber hohen Temperaturen empfindlich sind und anzunehmen war, daß die in den rohen Propinol-Lösungen vorhandenen Nebenbestandteile (Aldehyde und Acetale) unter dem Einfluß so hoher Temperaturen polymere Rückstände bilden und damit unerwünschte Ablagerungen auf dem Katalysator verursachen würden. Erstaunlicherweise wird aber die Lebensdauer des Katalysators bei der erfindungsgemäßen Hydrierung nicht beeinträchtigt. So behält z.B. der unter den Reaktionsbedingungen verwendeten Nickelkatalysator seine Aktivität bei, auch wenn er wiederholt für die gleiche Reaktion eingesetzt wird.

## Patentansprüche

1. Verfahren zur Herstellung von Propanol von hoher Reinheit durch Hydrierung von Propinol in wäßrigem Medium und in Gegenwart eines Hydrierkatalysators bei einem Wasserstoffpartialdruck von über 20 bar, dadurch gekennzeichnet, daß man die Hydrierung bei Temperaturen von über 200°C vornimmt.

2. Verfahren nach Anspruch 1, dadurch gekennzeichnet, daß man bei Temperaturen zwischen 210 und 280°C hydriert.

3. Verfahren nach Anspruch 1, dadurch gekennzeichnet, daß man die Hydrierung mit einem suspendierten Nickelkatalysator durchführt.

4. Verfahren nach Anspruch 1, dadurch gekennzeichnet, daß man zu dem zu hydrierenden wäßrigen Gemisch ein wasserlösliches Nickelsalz einer organischen Säure mit mindestens 2 C-Atomen gibt und nach der Hydrierung aus dem Reaktionsgemisch metallisches Nickel mechanisch abtrennt.

5. Verfahren nach Anspruch 1, dadurch gekennzeichnet, daß man bei Temperaturen von 230 bis 260°C und Wasserstoffpartialdrücken von 50 bis 250 bar hydriert.

6. Verfahren nach Anspruch 4, dadurch gekennzeichnet, daß man das abgetrennte Nickel durch Behandlung mit einer organischen Säure mit mindestens 2 C-Atomen in das Nickelsalz überführt und dieses direkt für die Hydrierung heranzieht.

## Claims

1. A process for the preparation of high-purity propanol by hydrogenating propynol in an aqueous medium and in the presence of a hydrogenation catalyst at a hydrogen partial pressure of greater than 20 bar, which comprises carrying out the hydrogenation at above 200°C.

2. A process as claimed in claim 1, wherein the hydrogenation is carried out at from 210 to 280°C.

3. A process as claimed in claim 1, wherein the hydrogenation is carried out using a suspended nickel catalyst.

4. A process as claimed in claim 1, wherein a watersoluble nickel salt of an organic acid having 2 or more carbon atoms is added to the aqueous mixture to be hydrogenated, and metallic nickel is separated mechanically from the reaction mixture after the hydrogenation.

5. A process as claimed in claim 1, wherein the hydrogenation is carried out at from 230 to 260°C and at a hydrogen partial pressure of from 50 to 250 bar.

6. A process as claimed in claim 4, wherein the separated nickel is converted into the nickel salt by treatment with an organic acid having 2 or more carbon atoms and the nickel salt is employed directly for the hydrogenation.

## Revendications

1. Procédé de préparation de propanol de pureté élevée par hydrogénation de propynol e milieu aqueux et en présence d'un catalyseur d'hydrogénation sous une pression partielle d'hydrogène supérieure à 20 bar, caractérisé en ce qu'on méne l'hydrogénation à des températures de plus de 200°C.

2. Procédé selon la revendication 1, caractérisé en ce qu'on hydrogène à des températures comprises entre 210 et 280ÉC.

3. Procédé selon la revendication 1, caractérisé en ce qu'on effectue l'hydrogénation avec un catalyseur de nickel en suspension.

4. Procédé selon la revendication 1, caractérisé en ce qu'on ajoute au mélange aqueux à hydrogéner un sel de nickel hydrosoluble d'un acide organique ayant au moins 2 atomes de carbone et on enlève mécaniquement du mélange réactionnel après hydrogénation le nickel métallique.

5. Procédé selon la revendication 1, caractérisé en ce qu'on hydrogène à des températures de 230 à 260°C et des pressions partielles d'hydrogène de 50 à 250 bars.

6. Procédé selon la revendication 4, caractérisé en ce qu'on transforme le nickel enlevé en sel de nickel par traitement par un acide organique ayant au moins 2 atomes de carbone et on utilise ce sel directement pour l'hydrogénation.